# EUROPEAN PATENT APPLICATION

(11) **EP 2 123 176 A1**
(43) Date of publication of application: **25.11.2009**
(21) Application number: 08156555.8
(22) Date of filing: 20.05.2008
(51) Int. Cl.: A23L 1/227, C07C 233/47, C07C 233/49

(54) **Acylamino acid compounds and food preparations containing same**

(71) Applicant: Nestec S.A., 1800 Vevey (CH)
(72) Inventor: Riera, Céline, 1012 Lausanne (CH); Menozzi, Candice, 1009 Pully (CH); Robert, Fabien, 01220 Divonne les Bains (FR); Le coutre, Johannes, 1009 Pully (CH)
(74) Representative: Rupp, Christian

(57) **Abstract**

An acylamino acid compound is described which can be used in food preparations to generate a savory tingling sensation without burning effect.

## Description

### Field of the Invention

The present invention relates to the culinary field. The present invention relates in particular to acylamino acid compounds or a salt thereof and their use in food preparations to generate a savory tingling sensation without burning effects. The present invention also relates to food preparations comprising an acylamino acid compound or a salt thereof.

### Background Art

Research on the molecular mechanisms underlying pungent sensations revealed the existence of two cation channels, TRPV1 (transient receptor potential V1) and TRPA1 (transient receptor potential A1) that are expressed in the somatosensory fibers innervating the oral cavity. TRPV1 is the receptor for heat and burning sensations such as capsaicin, the hot molecule in red hot chilli peppers. TRPA1 responds to cold and pungent compounds such as allyl isothiocyanate (mustard oil) and cinnamaldehyde (cinnamon). At moderated concentrations, TRPA1 agonists exhibit a pleasant tingling sensation.

Capsaicin causes a burning sensation when it comes in contact with mucous membranes. Thus, it is commonly used in food products to give them added spice or pungency.

However, food products containing red chilli peppers are frequently not accepted by the consumer as being too hot providing a very unpleasant mouth feeling. In particular, both the tingling and burning effect are considered to be very unsavory affecting the consumption of the food product.

It is therefore an object of the present invention to accommodate the needs of consumers which want to enjoy a spicy food without the interfering effect of burning sensations.

It is also an object of the present invention to provide a spicy food product which can be consumed without any side-effect such as burning sensations in the oral cavity.

### Summary of the Invention

Accordingly, this object is achieved by means of the features of the independent claims. The dependent claims further define preferred embodiments of the present invention.

The present invention describes, in a first aspect, fatty acid based acylamino acid compounds having the formula 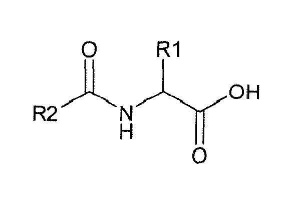 wherein R1 is selected from hydrogen, an alkyl group having 1 to 10 carbon atoms, a hydroxyalkyl group having 1 to 10 carbon atoms and a carboxyalkyl group having 1 to 10 carbon atoms and R2 is a straight chain hydrocarbon group having 5 to 20 carbon atoms and containing at least one double bond, or a salt thereof.

The compounds of the present invention generate new sensory properties. Diverse reactivity properties in vitro of these compounds have been found. More importantly, the acylamino acid compounds of the present invention exert a taste mainly tingling and not burning in the oral cavity when added to a food preparation. The compounds of the present invention are major TRPA1 agonists which have only a minor effect on TRPV1.

According to a second aspect of the invention, the present invention provides food preparations comprising an acylamino acid compound or a salt thereof sufficient to generate mainly tingling and not burning sensations in the oral cavity.

Finally, according to a third aspect of the invention, the amino acid compound or a salt is used for generating a mainly tingling and not burning sensation in a food preparation.

The invention will now be described in more detail by means of illustrative embodiments.

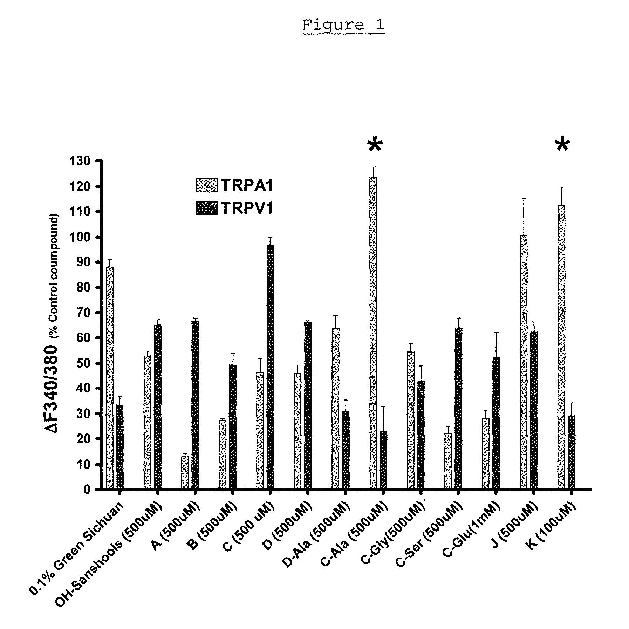

### Brief Description of the Drawing

The figure shows the effect of the compounds of the present invention on TRPA1 and TRPV1.

### Detailed Description of the present Invention

The compounds of the present invention are products based on fatty acids and aminoacids which can be either naturally occurring or chemically modified. Both structural units are linked by a coupling reaction.

It is essential to the compounds of the present invention that at least one cis double bond is present in the straight chain hydrocarbon group having 5 to 20 carbon atoms. This structural feature induces reactivity on TRPA1 providing a mainly tingling taste. At the same time, TRPV1 is only moderately stimulated so that a burning sensation is substantially suppressed.

In a preferred embodiment of the present invention, the alkyl group in the above formula comprises 1 to 5 carbon atoms, more preferred 1 to 3 carbon atoms.

The compounds of the present invention comprise preferably 1 to 5 carbon atoms, more preferred 1 to 3 carbon atoms.

In a further preferred embodiment of the present invention, the carboxyalkyl group in the above formula com-prises 1 to 5 carbon atoms, more preferred 1 to 3 carbon atoms.

It has been shown that the straight chain hydrocarbon group contained in the compounds of the present invention preferably comprises 10 to 18 carbon atoms, with 11 to 17 carbon atoms being preferred.

Particularly preferred examples of the acylamino acid compounds of the present inventions are those, wherein, in the formula, R1 is selected from hydrogen, methyl, hydroethyl and carboxyl ethyl and the straight chain hydrocarbon group comprises 11 carbon atoms.

The salts of the acylamino acid compounds of the present invention are preferably those usually used in the food industry. Examples for the salts are chlorides, sulfates, phosphates, gluconates, sodium, citrates, carbonates, acetates, lactates.

The figure shows a diagram illustrating test experiments using the compounds of the present invention on the ion channels TRPA1 and TRPV1. The following compounds have been tested including the reference compounds:
green Sichuan which is the extract of Sichuan pepper; OH-sanshools (major compound in Sichuan pepper responsible for the tingling and burning tastes);
synthetic analogues of α-hydroxy-sanshool A, B, C and D, the chemical formulae thereof are shown below;
C-Ala which is the carboxylic acid derivative of C coupled to alanine;
C-Gly which is the carboxylic acid derivative of C coupled to glycine;
C-Ser which is the carboxylic acid derivative of C coupled to serine;
C-Glu which is the carboxylic acid derivative of C coupled to glutamine;
D-Ala being the carboxylic acid derivative of D condensed with alanine; and
Compounds J and K are two natural fatty acids, i.e., palmitoleic and linolenic acids coupled with alanine.

Synthetic analogues A, B, C and D are derived from α-hydroxy-sanshool and have the following formulae:

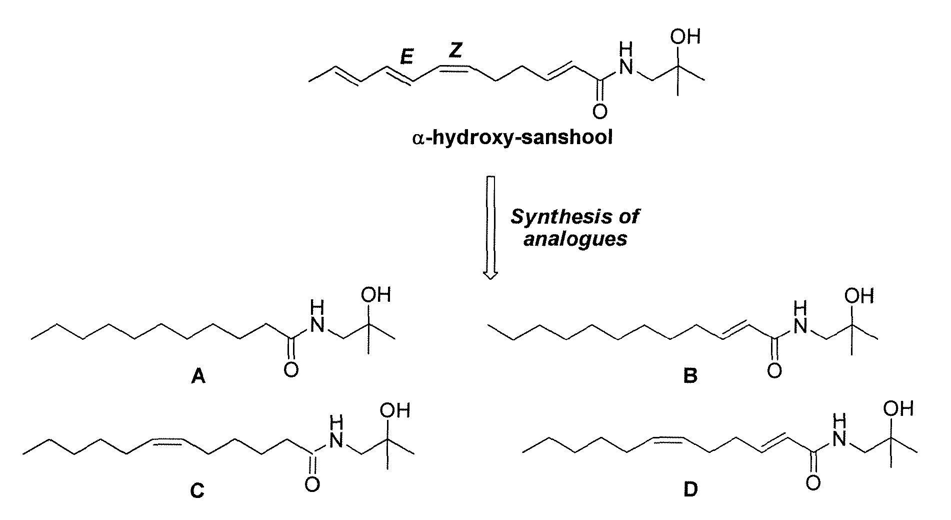

As can be seen from the maximum activation on TRPA1 and TRPV1 observed for saturating concentration of the compounds of the present invention, TRPA1 has been shown to be strongly stimulated while TRPV1 was purely activated. In particular, C-Ala, compounds J and K showed a strong stimulating effect on TRPA1, while C-Ala and K are purely activating TRPV1.

The compounds of the present invention can be synthesized following methods common in organic chemistry. A synthesis example is a coupling reaction using, as a starting material, a fatty acid and an amino acid to obtain the acylamino acid compound of the present invention. In a preferred embodiment, the coupling reaction is a condensation reaction. The fatty acids as well as the amino acid are selected from natural occurring or chemically modified fatty acids or amino acids.

The food preparation according to the present invention comprises an acylamino acid compound or a salt thereof in an amount sufficient to generate mainly tingling and not burning sensations in the oral cavity. In a preferred embodiment of the present invention, the food preparation comprises an acylamino acid compound having the following formula: 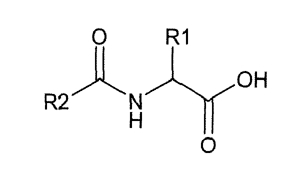 wherein R1 is selected from hydrogen, an alkyl group having 1 to 10 carbon atoms, a hydroxyalkyl group having 1 to 10 carbon atoms and a carboxyalkyl group having 1 to 10 carbon atoms and R2 is a straight chain hydrocarbon group having 5 to 20 carbon atoms and containing at least one double bond, or a salt thereof.

The content of the acylamino acid compound or a salt thereof in the food preparation is usually in a range of 10 micrograms to 500 milligrams per kilogram.

In the above formula, the alkyl group preferably comprises 1 to 5 carbon atoms, the hydroxyalkyl group comprises 1 to 5 carbon atoms, the carboxyalkyl group comprises 1 to 5 carbon atoms and the straight chain hydrocarbon group comprises 10 to 18 carbon atoms.

More preferred, in the above formula, the alkyl group preferably comprises 1 to 3 carbon atoms, the hydroxyalkyl group comprises 1 to 3 carbon atoms, the carboxyalkyl group comprises 1 to 3 carbon atoms and the straight chain hydrocarbon group comprises 11 to 17 carbon atoms.

In a particular preferred embodiment of the invention, the food preparation comprises an acylamino acid wherein, in the formula, R1 is selected from hydrogen, methyl, hydroxyl ethyl and carboxyl ethyl and the strain chain hydrocarbon group comprises 11 carbon atoms.

The compounds of the present invention are particularly applicable to food preparations which demand for a delicate hot sensation in the mouth cavity. Examples of food preparations are food additives, food products such as beverages, soups, ice-cream, confectionary, dairy, petfood and neutraceuticals.

In an alternative embodiment of the present invention, the acylamino acid "alpha-carboxyamide" compound is formed in situ from the condensation of fatty acids and amino acids available in the food preparation via thermal treatment followed by a basic hydrolysis and final neutralisation.

It has been shown that replacing the carboxyl terminus of the fatty acid by an amino acid potentiated dramatically the effect on TRPA1 and abolished the activity on TRPV1. The compounds oft the present invention used in food preparations have been surprisingly shown to provide mainly tingling sensations in the mouth cavity without exhibiting the unpleasant accompanying burning sensation usually occurring with the extract of Sichuan pepper.

According to the invention, the acylamino acid compound or a salt thereof as presented above is used for generating a mainly tingling and not burning sensation in a food preparation. The compounds of the present invention are preferably used as a spice, flavour or seasoning.

## Claims

1. An acylamino acid compound having the formula 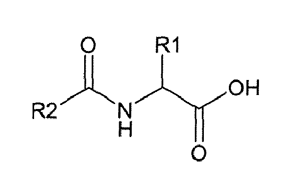 wherein R1 is selected from hydrogen, an alkyl group having 1 to 10 carbon atoms, a hydroxyalkyl group having 1 to 10 carbon atoms and a carboxyalkyl group having 1 to 10 carbon atoms and R2 is a straight chain hydrocarbon group having 5 to 20 carbon atoms and containing at least one double bond, or a salt thereof.

2. The compound according to claim 1 wherein, in the formula, the alkyl group comprises 1 to 5 carbon atoms.

3. The compound according to claim 1 and/or claim 2 wherein, in the formula, the hydroxyalkyl group comprises 1 to 5 carbon atoms.

4. The compound according to any of claims 1 to 3 wherein, in the formula, the carboxyalkyl group comprises 1 to 5 carbon atoms.

5. The compound according to any of claims 1 to 4 wherein, in the formula, the straight chain hydrocarbon group comprises 10 to 18 carbon atoms.

6. The compound according to any of claims 1 to 5 wherein, in the formula, R1 is selected from hydrogen, methyl, hydroxyethyl and carboxyethyl, and the straight chain hydrocarbon group comprises 11 carbon atoms.

7. A food preparation comprising an acylamino acid compound or a salt thereof in amount sufficient to generate mainly tingling and not burning sensations in the oral cavity.

8. The food preparation according to claim 7 wherein the acylamino acid compound has the following formula: 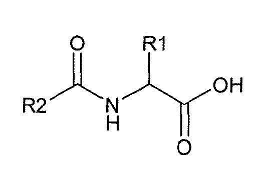 wherein R1 is selected from hydrogen, an alkyl group having 1 to 10 carbon atoms, a hydroxyalkyl group having 1 to 10 carbon atoms and a carboxyalkyl group having 2 to 10 carbon atoms and R2 is a straight chain hydrocarbon group having 5 to 20 carbon atoms and containing at least one double bond, or an salt thereof.

9. The food preparation according to claim 8 wherein, in the formula, the alkyl group comprises 1 to 5 carbon atoms, the hydroxyalkyl group comprises 1 to 5 carbon atoms, the carboxyalkyl group comprises 1 to 5 carbon atoms and the straight chain hydrocarbon group comprises 10 to 18 carbon atoms.

10. The food preparation according to claim 9 wherein, in the formula, R1 is selected from hydrogen, methyl, hydroxyethyl and carboxyethyl, and the straight chain hydrocarbon group comprises 11 carbon atoms.

11. The food preparation according to any of claims 7 to 10 wherein the acylamino acid compound is formed in situ by condensation from fatty acids and amino acids available in the food preparation.

12. Use of an acylamino acid compound or a salt thereof for generating a mainly tingling and not burning sensation in a food preparation.

13. The use of claim 12 wherein the acylamino acid compound or a salt thereof comprises the formula 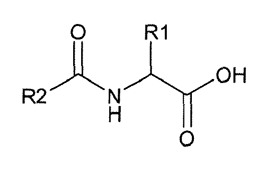 wherein R1 is selected from hydrogen, an alkyl group having 1 to 10 carbon atoms, a hydroxyalkyl group having 1 to 10 carbon atoms and a carboxyalkyl group having 1 to 10 carbon atoms and R2 is a straight chain hydrocarbon group having 5 to 20 carbon atoms and containing at least one double bond, or an salt thereof.

14. The use according to claim 13 wherein, in the formula, the alkyl group comprises 1 to 5 carbon atoms, the hydroxyalkyl group comprises 1 to 5 carbon atoms, the carboxyalkyl group comprises 1 to 5 carbon atoms and the straight chain hydrocarbon group comprises 10 to 18 carbon atoms.

15. The use according to any one of the claims 12 to 14 as a spice, flavour or seasoning.
